# EUROPEAN PATENT SPECIFICATION

(11) **EP 1 641 456 B1**
(45) Date of publication and mention of the grant of the patent: **03.03.2010**
(21) Application number: 04738956.4
(22) Date of filing: 25.06.2004
(51) Int. Cl.: A61K 31/437, A61K 31/445, A61K 31/343, A61K 31/4439, A61K 31/404, A61K 31/15, A61P 25/18

(54) **GABOXADOL FOR TREATING DEPRESSION AND OTHER AFFECTIVE DISORDERS**
GABOXADOL ZUR BEHANDLUNG VON DEPRESSIONEN UND ANDEREN AFFEKTIVEN STÖRUNGEN
TRAITEMENT DE LA DEPRESSION ET D'AUTRES TROUBLES AFFECTIFS

(30) Priority: 25.06.2003 DK 200300956; 25.06.2003 US 483019 P; 07.01.2004 US 535123 P; 07.01.2004 DK 200400016
(43) Date of publication of application: 05.04.2006
(60) Divisional application: 09173892.2
(73) Proprietor: H. LUNDBECK A/S, 2500 Valby-Copenhagen (DK)
(72) Inventor: SANCHEZ, Connie, West Milford, 07480 NJ (US); EBERT, Bjarke, DK-3520 Farum (DK)
(86) International application number: PCT/DK2004/000459
(87) International publication number: WO 2004/112786

(56) References cited:
- EP-A- 1 254 668
- CHRISTENSEN A V ET AL: "Pharmacodynamic effects and possible therapeutic uses of THIP, a specific GABA-agonist." PHARMACEUTISCH WEEKBLAD. SCIENTIFIC EDITION. 22 OCT 1982, vol. 4, no. 5, 22 October 1982 (1982-10-22), pages 145-153, XP008035614 ISSN: 0167-6555
- PETERSEN ET AL.: ACTA NEUROL SCAND., vol. 67, 1983, pages 114-117,
- KORSGAARD ET AL: ARCH GEN PSYCHIATRY, vol. 39, 1982, pages 1017-1021,
- MOHR ET AL CLINICAL NEUROPHARMACOLOGY vol. 9, pages 257 - 263
- FOSTER ET AL: NEUROLOGY, vol. 33, 1983, pages 637-639,

## Description

### Field of invention

The present invention relates to the use of gaboxadol as monotherapy for the preparation of medicaments useful for the treatment of depression.

### Background of the Invention

Gaboxadol (THIP), described in EP patent 0000338 B1 and in EP Patent 0840601 B1, have shown great potential in the treatment of sleep disorders.

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression and anxiety disorders indicate that non-response to SSRIs is substantial, up to 30%. Another, often neglected, factor in antidepressant treatment is compliance, which has a rather profound effect on the patient's motivation to continue pharmacotherapy.

EP 1 254 668 A discloses a combination of a serotonin reuptake inhibitor and a GABA-A alpha 2/3 agonist, selected from gaboxadol, ganaxolone, fengabine, 2-(4-methoxyphenyl)-2,5,6,7,8,9-hexahydro-pyrazolo[4,3-c]cinnolin-3-one, 7-cyclobutyl-6-(2-methyl-2H-1,2,4-triazol-3-ylmethoxy)-3-phenyl-1,2,4-triazolo[4,3-b]pyridazine, (3-fluoro-4-methylphenyl)-N-({1-[(2-methylphenyl)methyl]-benzimidazol-2-yl}methyl)-N-pentylcarboxamide and 3-(aminomethyl)-5-methylhexanoic acid, for use in anxiety and depression.

Christensen A V et al: "Pharmacodynamic effects and possible therapeutic uses of THIP, a specific GABA-agonist", Pharmaceutisch Weekblad, Scientific Edition, 22 Oct 1982, vol 4, no. 5, 22 October 1982, pages 145-153, discloses that the specific GABA agonistic profile of THIP makes it likely that THIP could be of beneficial value in diseases where evidence of GABA-ergic hypofunction has been obtained. Furthermore Christensen et al. discloses that there is a long list of diseases where such evidence has been obtained: epilepsy, tardive dyskinesia, spasticity, parkinsonism, Huntingdon's chorea, Alzheimer's disease, depression, and schizophrenia.

### Description of the Invention

According to the present invention a pharmaceutical composition for the treatment of depression is provided.

Gaboxadol has the general formula 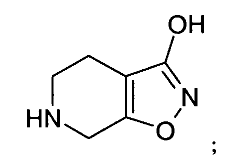 and throughout the description "gaboxadol" is intended to include any form of the compound, such as the base (zwitter ion), pharmaceutically acceptable salts, e.g. pharmaceutically acceptable acid addition salts, hydrates or solvates of the base or salt, as well as anhydrates, and also amorphous, or crystalline forms.

More specifically, the present invention relates to the use of gaboxadol as monotherapy having the general formula 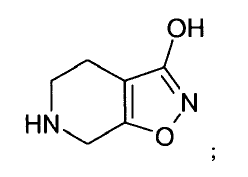 for the preparation of a pharmaceutical composition for the treatment of depression.

In a further aspect, the present invention relates to use of gaboxadol as monotherapy for preparing a pharmaceutical composition for treating depression in an individual. Such individual is preferably a human, such as male or female human, child, adult or elderly.

According to the invention, gaboxadol may be used as the base (the zwitter ion) or as a pharmaceutically acceptable acid addition salt thereof or as an anhydrate or hydrate of such salt or base. The salts of the compound used in the invention are salts formed with non-toxic organic or inorganic acids. Exemplary of such organic salts are those with maleic, fumaric, benzoic, ascorbic, succinic, oxalic, bis-methylenesalicylic, methanesulfonic, ethane-disulfonic, acetic, propionic, tartaric, salicylic, citric, gluconic, lactic, malic, mandelic, cinnamic, citraconic, aspartic, stearic, palmitic, itaconic, glycolic, p-amino-benzoic, glutamic, benzene sulfonic and theophylline acetic acids, as well as the 8-halotheophyllines, for example 8-bromo-theophylline. Exemplary of such inorganic salts are those with hydrochloric, hydrobromic, sulfuric, sulfamic, phosphoric and nitric acids. Gaboxadol may also be used as the zwitter ion, e.g. the mono hydrate thereof.

The acid addition salts according to the invention may be obtained by treatment of gaboxadol with the acid in an inert solvent followed by precipitation, isolation and optionally re-crystallisation by known methods and if desired micronisation of the crystalline product by wet or dry milling or another convenient process, or preparation of particles from a solvent-emulsification process. Suitable methods are described in EP patent 0000338.

Precipitation of the salt is typically carried out in an inert solvent, e.g. an inert polar solvent such as an alcohol (e.g. ethanol, 2-propanol and n-propanol), but water or mixtures of water and inert solvent may also be used.

In an embodiment, gaboxadol is used in the form of an acid addition salt, or a zwitter ion hydrate or zwitter ion anhydrate. In a further embodiment, gaboxadol is used in the form of the pharmaceutically acceptable acid addition salt selected from the hydrochloride or hydrobromide salt, or in the form of the zwitter ion monohydrate. Most conveniently, gaboxadol is in a crystalline form.

According to the invention, gaboxadol may be administered in any suitable way e.g. orally or parenterally, and it may be presented in any suitable form for such administration, e.g. in the form of tablets, capsules, powders, syrups or solutions or dispersions for injection. Preferably, and in accordance with the purpose of the present invention, gaboxadol is administered in the form of a solid pharmaceutical entity, suitably as a tablet or a capsule or in the form of a suspension, solution or dispersion for injection.

Methods for the preparation of solid pharmaceutical preparations are well known in the art. Tablets may thus be prepared by mixing the active ingredients with ordinary adjuvants and/or diluents and subsequently compressing the mixture in a convenient tabletting machine. Examples of adjuvants or diluents comprise: corn starch, lactose, talcum, magnesium stearate, gelatine, lactose, gums. Any other adjuvant or additive such as colourings, aroma, preservatives, may also be used provided that they are compatible with the active ingredients.

A suitable formulation of gaboxadol is described in WO 02/094225 filed May 17, 2002.

Gaboxadol is typically administered as an oral dose form, such as a solid oral dose form, typically tablets or capsules, or as a liquid oral dose form. Gaboxadol is most conveniently administered orally in unit dosage forms such as tablets or capsules, containing the active ingredient in an amount from about 0.1 to about 150 mg/day, such as from about 0.1 to about 100 mg/day, typically from about 0.5 to about 50 mg/day, preferably from about 2.5 to about 20 mg/day, e.g. from about 5 to about 15 mg/day. The amount of gaboxadol is calculated based on the free base form.

Gaboxadol is administered as monotherapy.

Typically, gaboxadol is used in the form of an acid addition salt, or a zwitter ion hydrate or zwitter ion anhydrate. In a further embodiment, gaboxadol is used in the form of the pharmaceutically acceptable acid addition salt selected from the hydrochloride or hydrobromide salt, or in the form of the zwitter ion monohydrate. Most conveniently, gaboxadol is in a crystalline form.

To prepare the pharmaceutical compositions of this invention, an appropriate amount of the active ingredient(s), in salt form or base form, is combined in an intimate admixture with a pharmaceutically acceptable carrier, which can take a wide variety of forms depending on the form of preparation desired for administration. These pharmaceutical compositions are desirably in unitary dosage form suitable for administration orally, rectally, percutaneously or by parenteral injection. For example, in preparing the compositions in oral dosage form, any of the usual pharmaceutical media may be employed, such as, for example, water, glycols, oils, alcohols in the case of oral liquid preparations such as suspensions, syrups, elixirs and solutions; or solid carriers such as starches, sugars, kaolin, lubricants, binders, disintegrating agents in the case of powders, pills, capsules and tablets. Because of their ease in administration, tablets and capsules represent the most advantageous oral dosage unit form, in which case solid pharmaceutical carriers are obviously employed.

It is especially advantageous to formulate the aforementioned pharmaceutical compositions in dosage unit form for ease of administration and uniformity of dosage. As used in the specification and claims, unit dosage form refers to physically discrete units suitable as unitary dosages, each unit containing a predetermined quantity of active ingredient(s) calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls, and segregated multiples thereof.

### Pharmacological Test of antidepressant effect of gaboxadol

The rat chronic mild stress (CMS) model of depression has a high degree of preditive validity for antidepressant activity (Willner (1997), Psychopharmacol 134:319-329). Furthermore the procedure is an appropriate model to study onset of antidepressant action in animals. (Behavioural Pharmacology 14:465-470, 2003, Sánchez, C. et al). The principle of the model is based on the relation between stress and affective disorders. Rats exposed to chronic stress will show a reduced sensitivity to rewards (e.g. a palatable sucrose solution).

### Experimental Procedure chronic mild stress

Male Wistar rats (Gorzkowska, Warsaw) were brought into the laboratory two months before the start of the experiment. The animals were singly housed with food and water freely available, and maintained on a 12-h light/dark cycle at a temperature of 22 ± 2 °C, except as described below.

The animals were first trained to consume a 1% sucrose solution; training consisted of eight 1h baseline tests in which sucrose was presented, in the home cage, following 14h food and water deprivation; intake was measured by weighing pre-weighed bottles containing the sucrose solution, at the end of the test. Subsequently, sucrose consumption was monitored, under similar conditions, throughout the whole experiment. On the basis of their sucrose intakes in the fmal baseline test, the animals were divided into two matched groups. One group of animals was subjected to a chronic mild stress procedure for a period of 8 consecutive weeks. The weekly stress regime consisted of 2 periods of food or water deprivation, 45 degree cage tilt, intermittent illumination (lights on and off every 2 h), soiled cage (250 ml water in sawdust bedding), paired housing and low intensity stroboscopic illumination (150 flashes/min), and 2 periods of no stress. All stressors were 10 - 14 h of duration and were applied individually and continuously, day and night. The other groups of animals, the unstressed controls, were housed in separate rooms and had no contact with the stressed animals. The rats were deprived of food and water for the 14 h preceding each sucrose test, but otherwise food and water were freely available in the home cage. On the basis of their sucrose intake scores following 3 weeks of stress, both stressed and control animals were divided into matched subgroups, and for the subsequent five weeks they received twice daily intraperitoneal drug injections (at approximately 10.00 and 17.00). Sucrose tests were performed at 10 a.m. on a weekly basis (Tuesdays). Different groups of animals (n = 8) were administered vehicle (1 ml/kg per day) or test drug. Before test sessions drugs were administered at approximately 10.00h and the sucrose tests were carried out 24h following the previous drug injection. Stress was continued throughout the entire treatment period. Body weights were assessed a baseline and by the end of drug treatment.

### Results chronic mild stress

The testing of gaboxadol in the rat chronic mild stress model showed that the compound had a significant anti-depressant-like effect. The dose of 2.5 mg/kg per day had a clear and significant effect in this model.

### Gaboxadol in combination with escitalopram - reference examples

In order to modulate the activity of systems, responsible for antidepressants activity, we have characterized the interaction between gaboxadol and escitalopram (an SSRI) in a behavioural model of serotonin reuptake inhibition, the mouse 5-HTP potentiation test, and a model that is predictive of antidepressant activity, the mouse forced swim test (cf. C. Sánchez et al, Psychopharmacology (2003), 167:353-362).

### Experimental Procedures

Male NMRI/BOM mice (18-25 g; Bomholtgaard, Denmark) were used. The mice were housed in plastic cages (35 x 30 x 12 cm), 10 in each and habituated to the animal facilities for at least a week before test. The room temperature (21 ± 2°C), relative humidity (55 ± 5%), and air exchange (16 times per h) were automatically controlled. The animals had free access to commercial food pellets and tap water before test.

### Potentiation of 5-HTP-induced behaviour.

The test procedure for studies in mice is described in detail by Hyttel et al (1992). Briefly, 30 min after s.c. administration of test compound mice were given 5-HTP (100 mg/kg, i.v.). Thereafter the animals were evaluated in their home cage during a 15-min observation period with respect to stereotypy (lateral head movements), tremor, and hind limb abduction. One point was given for each symptom being present. A total of 8-16 mice were used per dose.

### Inhibition of forced swimming-induced immobility.

A mouse that is forced to swim in a spatially constrained container will exert a characteristic immobile posture. Pretreatment with an antidepressant will counteract this effect. The test was conducted as described in detail by Sánchez and Meier (Psychopharmacol 129:197-205; 1997). Briefly, a fully automated test system with 6 swim units (2000 ml glass jars filled with 1200 ml soiled water (23 - 25°C) in which a mouse had been placed previously) was used. The assessment of immobility was performed by image analysis. Thirty minutes after drug or vehicle treatment the mouse was placed into the glass jar and left in the water for a total of 6 min. The accumulated duration of immobility was measured during the last 3 min. A total of 9-18 mice were tested per dose.

### Results

Gaboxadol (2.5 mg/kg) strongly potentiated the acute effects of escitalopram (0.5 to 0.025 mg/kg) in the 5-HTP potentiation test and a gaboxadol dose (2.5 mg/kg) that was not active by itself in the forced swim test potentiated the antidepressant-like effect of escitalopram (2.5 and 5 mg/kg) significantly.

## Claims

1. Use of gaboxadol as monotherapy for preparing a pharmaceutical composition for treating depression wherein the pharmaceutical composition comprises from 2.5 mg to 20 mg, such as 5 mg to 15 mg of gaboxadol.

2. The use of claim 1 wherein gaboxadol is in the form of an acid addition salt, or a zwitter ion hydrate or zwitter ion anhydrate.

3. The use of any one of claims 1-2 wherein gaboxadol is in the form of a pharmaceutically acceptable acid addition salt selected from the hydrochloride or hydrobromide salt, or in the form of the zwitter ion monohydrate.

4. The use of any one of claims 1-3 wherein gaboxadol is in an oral dose form.

5. The use of any one of claims 1-4 wherein gaboxadol is in a solid oral dose form, such as tablets or capsules, or a liquid oral dose form.

6. The use of any one of claims 1-5 wherein said gaboxadol is crystalline.

7. The use of any one of claims 1-6 wherein said pharmaceutical composition is for treating a human having a depression.

## Patentansprüche

1. Verwendung von Gaboxadol als Monotherapie zur Herstellung einer pharmazeutischen Zusammensetzung zur Behandlung von Depression, worin die pharmazeutische Zusammensetzung von 2,5 mg bis 20 mg, wie 5 mg bis 15 mg Gaboxadol enthält.

2. Verwendung nach Anspruch 1, worin Gaboxadol in der Form eines Säureadditionssalzes oder eines Zwitterionenhydrates oder Zwitterionenanhydrates vorliegt.

3. Verwendung nach einem der Ansprüche 1 bis 2, worin Gaboxadol in der Form eines pharmazeutisch akzeptablen Säureadditionssalzes vorliegt, ausgewählt aus dem Hydrochlorid- oder Hydrobromidsalz oder in der Form des Zwitterionenmonohydrates.

4. Verwendung nach einem der Ansprüche 1 bis 3, worin Gaboxadol in einer oralen Dosierungsform vorliegt.

5. Verwendung nach einem der Ansprüche 1 bis 4, worin Gaboxadol in einer festen oralen Dosisform, wie Tabletten oder Kapseln, oder einer flüssigen oralen Dosisform vorliegt.

6. Verwendung nach einem der Ansprüche 1 bis 5, worin das Gaboxadol kristallin ist.

7. Verwendung nach einem der Ansprüche 1 bis 6, worin die pharmazeutische Zusammensetzung zur Behandlung eines Menschen mit einer Depression ist.

## Revendications

1. Utilisation de gaboxadol comme monothérapie pour la préparation d'une composition pharmaceutique destinée au traitement de la dépression, dans laquelle la composition pharmaceutique comprend de 2,5 mg à 20 mg, de préférence 5 mg à 15 mg de gaboxadol.

2. Utilisation selon la revendication 1, dans laquelle le gaboxadol est sous forme d'un sel d'addition d'acide, ou d'un hydrate de zwitterion ou d'un zwitterion anhydre.

3. Utilisation selon l'une quelconque des revendications 1-2, dans laquelle le gaboxadol est sous forme d'un sel d'addition d'acide pharmaceutiquement acceptable choisi parmi le sel de chlorhydrate ou de bromhydrate, ou sous forme du monohydrate de zwitterion.

4. Utilisation selon l'une quelconque des revendications 1-3, dans laquelle le gaboxadol est sous une forme galénique orale.

5. Utilisation selon l'une quelconque des revendications 1-4, dans laquelle le gaboxadol est sous une forme galénique orale solide, comme des comprimés ou des capsules, ou sous une forme galénique orale liquide.

6. Utilisation selon l'une quelconque des revendications 1-5, dans laquelle ledit gaboxadol est cristallin.

7. Utilisation selon l'une quelconque des revendications 1-6, dans laquelle ladite composition pharmaceutique est destinée au traitement d'un être humain ayant une dépression.
